# EUROPÄISCHE PATENTSCHRIFT

(11) **EP 2 951 154 B1**
(45) Veröffentlichungstag und Bekanntmachung des Hinweises auf die Patenterteilung: **19.04.2017**
(21) Anmeldenummer: 14701538.2
(22) Anmeldetag: 27.01.2014
(51) Int. Cl.: C07D 211/26, C07D 211/60

(54) **2,6-BIS-(AMINOMETHYL)PIPERIDIN- (2,6-BAMP-), 2,6-BIS-(ISOCYANOMETHYL)PIPERIDIN- (DIISOCYANAT-) UND 2,6-DICYANOPIPERIDIN-DERIVATE (2,6-DCP-DERIVATE) UND IHRE VERWENDUNG IN DER HERSTELLUNG VON EPOXYHARZEN, POLYURETHANEN, POLYETHEROLEN UND POLYAMIDEN**
2,6-BIS-(AMINOMETHYL)PIPERIDINE- (2,6-BAMP-), 2,6-BIS-(ISOCYANOMETHYL)PIPERIDINE- (DIISOCYANATE-) AND 2,6-DICYANOPIPERIDINE-DERIVATIVES (2,6-DCP-DERIVATIVES) AND THEIR USE IN THE PREPARATION OF EPOXY RESINS, POLYURETHANES, POLYETHEROLES AND POLYAMIDES
DÉRIVÉS DE 2,6-BIS-(AMINOMÉTHYL)PIPÉRIDINE (2,6-BAMP), 2,6-BIS-(ISOCYANOMÉTHYL)PIPÉRIDINE (DIISOCYANATES) ET 2,6-DICYANOPIPÉRIDINE (2,6-DCP) ET LEUR UTILISATION DANS LA PRODUCTION DE RÉSINS ÉPOXYDIQUES, POLYURÉTHANES, POLYÉTHEROLES ET POLYAMIDES

(30) Priorität: 30.01.2013 EP 13153262
(43) Veröffentlichungstag der Anmeldung: 09.12.2015
(73) Patentinhaber: BASF SE, 67056 Ludwigshafen am Rhein (DE)
(72) Erfinder: JAEGLI, Stephanie, 68163 Mannheim (DE); SCHMIDT, Thomas, 67433 Neustadt (DE); OFTRING, Alfred, 67098 Bad Dürkheim (DE); PANCHENKO, Alexander, 67071 Ludwigshafen (DE); DAHMEN, Kirsten, 67098 Bad Dürkheim (DE); MOLT, Oliver, 67269 Grünstadt (DE)
(86) Internationale Anmeldenummer: PCT/EP2014/051519
(87) Internationale Veröffentlichungsnummer: WO 2014/118121

(56) Entgegenhaltungen:
- EP-A1- 1 138 711
- WO-A1-2011/079041
- WO-A2-2010/009994
- DE-A1- 2 640 408

## Beschreibung

Die vorliegende Erfindung betrifft 2,6-bis-(Aminomethyl)piperidin-Derivate als solche (im nachfolgenden Text als "2,6-BAMP-Derivate" abgekürzt), die über die im nachfolgenden Text aufgeführte allgemeine Formel (I) definiert sind. Zudem betrifft die vorliegende Erfindung ein Verfahren zur Herstellung von solchen 2,6-BAMP-Derivaten durch Hydrierung der entsprechenden 2,6-Dicyanopiperidin-Derivate (nachfolgend als "2,6-DCP-Derivate" abgekürzt) in Gegenwart eines Katalysators. Die vorliegende Erfindung betrifft weiterhin die Verwendung der erfindungsgemäßen 2,6-BAMP-Derivate als Härter für Epoxyharze, als Zwischenprodukt bei der Herstellung von Diisocyanaten - die bei der Herstellung von Polyurethanen eine wichtige Rolle spielen -, als Starter bei der Herstellung von Polyetherolen und/oder als Monomer für die Polyamidherstellung. Die vorliegende Erfindung betrifft weiterhin die aus den 2,6-BAMP-Derivaten hergestellten Diisocyanate als solche sowie das entsprechende Herstellungsverfahren.

Es ist generell bekannt, dass aliphatische Nitrile in Gegenwart von Wasserstoff und Katalysatoren zu den entsprechenden Aminen hydriert werden können. Solche Hydrierverfahren sind sowohl für β-Aminonitrile als auch für diverse α-Aminonitrile, wie Aminoacetonitril (AAN) oder Ethylendiamindiacetonitril (EDDN), zur Herstellung der entsprechenden Amine, wie Ethylendiamin (EDA) oder Triethylentetramin (TETA), bekannt. Weiterhin ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung des eingesetzten Nitrils bzw. der H₂N-C-Bindung des durch die Hydrierung erhaltenen Amins. Das "Handbook of Heterogeneous Catalytic Hydrogenation for Organic Synthesis" (John Wiley & Sons, New York, 2001, Seiten 173-275) zeigt die Problematik der Hydrierung von α-Aminonitril anhand von zyklischen α-Aminonitrilen wie 1-Cyclohexyl-2,5-dicyano-2,5-dimethylpyrrolidin.

WO 2008/104553 betrifft ein Verfahren zur Herstellung von Triethylentetramin (TETA), wobei Ethylendiamindiacetonitril (EDDN) in Gegenwart eines Katalysators und eines Lösungsmittels hydriert wird. Weiterhin kann EDDN auch als Bestandteil eines Aminonitrilgemisches vorliegen, das zusätzlich Ethylendiaminmonoacetonitril (EDMN) enthält, wobei aus EDMN durch Hydrierung Diethylentriamin (DETA) erhalten wird. Bei TETA und DETA handelt es sich in beiden Fällen um nichtzyklische (lineare) Ethylenamine.

WO 2010/009995 betrifft 5-Isopropyl-3-aminomethyl-2-methyl-1-amino-cyclohexan (Carvondiamin) sowie ein Verfahren zu dessen Herstellung. Ausgehend von Carvon wird durch eine mehrstufige Reaktion mit Cyanwasserstoff und Ammoniak ein nitrilhaltiges Zwischenprodukt gebildet, das wiederum mit Wasserstoff zu Carvondiamin hydriert wird. Bei Carvondiamin handelt es sich um einen zyklischen Aliphaten (Cyclohexanderivat), der am Ring direkt mit einer ersten Aminofunktion sowie über eine Methylenbrücke mit einer weiteren Aminofunktion substituiert ist. Carvondiamin ist also im Gegensatz zu den erfindungsgemäßen 2,6-BAMP-Derivaten kein auf einem Heterocyclus basierendes Ethylenamin mit drei über zwei Ethylenbrücken verbundenen Aminofunktionen, sondern ein cycloaliphatisches Diamin mit zwei primären, sich ausschließlich in den Substituenten befindenden Aminofunktionen. Carvondiamin kann beispielsweise als Härter bei Epoxyharzen oder als Zwischenprodukt bei der Herstellung von Diisocyanaten eingesetzt werden.

WO 2010/009994 A2 offenbart 3-Aminomethyl-cyclohexylamin und eine Methode für dessen Herstellung. Es wird a) durch Umsetzung von Cyclohexenon mit Cyanwasserstoff in der Gegenwart eines basischen Katalysators, b) durch Umsetzung des in Stufe a) erhaltenen Cyclohexanonenitrils mit Ammoniak in der Gegenwart eines Iminbildungskatalysators , und c) Umsetzung der in Stufe b) gewonnenen 3-Cyanocyclohexylimin-haltigen Reaktionsmischung, mit Wasserstoff und Ammoniak an Hydrierungskatalysatoren.

EP- 1 138 711 A1 offenbart verschiedene monocyclische, heterocyclische Verbindungen, die zumindest ein NH₂ - substituiertes Ringstickstoffatom und keine CH₂-NH₂ Subtituenten in 2,6- position umfassen.

DE 2640408 A1 offenbart 2,2,6,6 Tetramethyl-4-amino-piperidin Härter für 1,2-Epoxidverbindungen.

WO 2011/079041 betrifft ein Verfahren zur Synthese von polycyclischen Guanidinverbindungen ausgehend von Triaminen. Ein Piperidinderivat wird lediglich als mögliches Ausgangsmaterial erwähnt, nicht aber dessen Synthese.

Die im erfindungsgemäßen Verfahren zur Herstellung der 2,6-BAMP-Derivate eingesetzten entsprechenden 2,6-DCP-Derivate sind bereits bekannt. So beschreibt beispielsweise GB-A 915 227 die Herstellung von 2,6-Dicyanopiperidin als solchem (auf die vorliegende Erfindung übertragen ist in der nachfolgenden Formel (II) der Rest R¹ also Wasserstoff und A, B, D sind jeweils CH₂), wobei Glutaraldehyd mit Blausäure (HCN) und Ammoniak umgesetzt wird. Eine etwaige Hydrierung des bei diesem Verfahren erhaltenen Produkts unter Erhalt der entsprechenden Aminomethylverbindung ist in diesem Dokument jedoch nicht offenbart. Im Stand der Technik sind noch zahlreiche weitere Dokumente bekannt, die allesamt Herstellungsverfahren von 2,6-DCP-Derivaten beschreiben, wie beispielsweise CHEM. REV. 1983 (4), Seiten 379-423.

Wie vorstehend im Zusammenhang mit WO 2010/009995 bereits ausgeführt, können Verbindungen mit zwei primären Aminofunktionen ("Diamine") für zahlreiche Verwendungen eingesetzt werden, beispielsweise als Härter bei Epoxyharzen oder zur Herstellung von Diisocyanaten. Die Struktur des eingesetzten Diamins kann die Eigenschaften der aus den Diaminen hergestellten Polymermaterialien, wie Wetterbeständigkeit, Hydrolysebeständigkeit, Chemikalienbeständigkeit, Lichtbeständigkeit, elektrische sowie mechanische Eigenschaften, beeinflussen. Sie kann aber auch einen Einfluss auf die Verarbeitbarkeit und die Verarbeitung der Diamine zu den entsprechenden Polymermaterialien, beispielsweise der Aushärtung von Epoxyharzen, ausüben.

Die der vorliegenden Erfindung zugrundeliegende Aufgabe besteht somit in der Bereitstellung von weiteren Verbindungen, die zwei primäre Aminofunktionen aufweisen sowie ein entsprechendes Herstellungsverfahren von solchen Verbindungen. Gelöst wird die Aufgabe durch 2,6-bis-(Aminomethyl)piperidin-Derivate (2,6-BAMP-Derivat) gemäß der allgemeinen Formel (I), 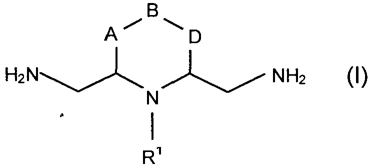 wobei:
- A, B und D: sind unabhängig voneinander CH₂, CHR² oder CR²R³;
- R¹: istunsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;

- R² und R³: sind unabhängig voneinander unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
- R⁴ und R⁵: sind unabhängig voneinander Wasserstoff oder unsubstituiertes C₁-C₄-Alkyl;
- R⁶: ist unsubstituiertes C₁-C₁₀-Alkyl.

Die erfindungsgemäßen 2,6-BAMP-Derivate können in vorteilhafter Weise bei hohem Umsatz und/oder hoher Selektivität hergestellt werden. Nebenprodukte, wie beispielsweise das entsprechende Mono-Aminomethylpiperidin-Derivat, das aus einer Hydrogenolyse einer der beiden C-CN-Bindungen bei der Dicyanedukthydrierung resultiert, fallen nur in geringen Mengen an bzw. können durch Steuerung von Verfahrensparametern wie Druck, Temperatur oder Katalysator weiter verringert werden. So kann beispielsweise das Verhältnis von N-Methyl-2,6-BAMP zum entsprechenden Monoaminomethyl-Nebenprodukt (N-Methyl-2-Aminomethylpiperidin; N-Methyl-2-BAMP) zwischen 1:1 bis 15:1 gesteuert werden. Insbesondere die Hydrierung von 2,6-DCP unter Erhalt des entsprechenden 2,6-BAMP als solchem (in der allgemeinen Formel (I) ist R¹ also Wasserstoff) kann mit hoher Selektivität von vorzugsweise 70 %, besonders bevorzugt > 85 %, durchgeführt werden.

Weiterhin ist es vorteilhaft, dass das Stereoisomerenverhältnis der 2,6-BAMP-Derivate gemäß der allgemeinen Formel (I) durch Einstellung der Reaktionsbedingungen reguliert werden kann. Sofern die als Edukte eingesetzten 2,6-DCP-Derivate aus Glutarsäure oder Glutarsäurederivate durch Umsetzung mit Blausäure und den entsprechenden Aminen hergestellt werden, können die Edukte bereits mit cis/trans-Verhältnissen im Bereich von 80:20 bis > 99:1 bereitgestellt werden. Diese cis/trans-Verhältnisse können bei der nachfolgenden Hydrierung zur Herstellung der erfindungsgemäßen 2,6-BAMP-Derivate aufrechterhalten werden.

Weiterhin können die erfindungsgemäßen 2,6-BAMP-Derivate in vorteilhafter Weise bei der Herstellung von Polymermaterialien, wie Epoxyharzen, Polyurethanen, Polyestern etc., eingesetzt werden, um das Eigenschaftsprofil dieser Polymermaterialien, beispielsweise in Hinblick auf Wetterbeständigkeit, Hydrolysebeständigkeit, Chemikalienbeständigkeit, Lichtbeständigkeit, elektrische und mechanische Eigenschaften, zu regulieren und erlauben somit höhere Variationsmöglichkeiten bei der Formulierung dieser Materialien.

Im Rahmen der vorliegenden Erfindung bedeuten Definitionen wie C₁-C₃₀-Alkyl, wie beispielsweise vorstehend für den Rest R¹ in Formel (I) definiert, dass dieser Substituent (Rest) ein Alkylrest mit einer Kohlenstoffatomanzahl von 1 bis 30 ist. Der Alkylrest kann sowohl linear als auch verzweigt sowie gegebenenfalls zyklisch sein. Alkylreste, die sowohl einen zyklische als auch eine lineare Komponente aufweisen, fallen ebenfalls unter diese Definition. Gleiches gilt auch für andere Alkylreste, wie beispielsweise ein C₁-C₄-Alkylrest oder ein C₁-C₁₀-Alkylrest. Beispiele für Alkylreste sind Methyl, Ethyl, n-Propyl, sek-Propyl, n-Butyl, sek-Butyl, iso-Butyl, 2-Ethylhexyl, tertiär-Butyl (tert-Bu/t-Bu), Pentyl, Hexyl, Heptyl, Cyclohexyl, Octyl, Nonyl oder Decyl.

Im Rahmen der vorliegenden Erfindung bedeuten Definitionen wie C₂-C₁₀-Alkenyl, wie beispielsweise vorstehend für den Rest R¹ in Formel (I) definiert, dass dieser Substituent (Rest) ein Alkenylrest mit einer Kohlenstoffatomanzahl von 2 bis 10 ist. Dieser Kohlenstoffrest ist vorzugsweise einfach ungesättigt, gegebenenfalls kann er aber auch zwei- oder mehrfach ungesättigt sein. Hinsichtlich Linearität, Verzweigungen sowie zyklischer Anteile gelten die sinngemäßen Angaben wie vorstehend anhand der C₁-C₃₀-Alkyl-Reste definiert. Vorzugsweise ist C₂-C₁₀-Alkenyl im Rahmen der vorliegenden Erfindung Vinyl, 1-Allyl, 3-Allyl, 2-Allyl, cis- oder trans-2-Butenyl, w-Butenyl.

Im Rahmen der vorliegenden Erfindung bedeutet der Begriff "Aryl" bzw. der Begriff "Aryl²", wie beispielsweise vorstehend für den Rest R¹ in Formel (I) definiert, dass der Substituent (Rest) ein Aromat ist. Bei dem Aromat kann es sich um einen monozyklischen, bizyklischen oder gegebenenfalls polyzyklischen Aromaten handeln. Im Fall von polyzyklischen Aromaten können gegebenenfalls einzelne Zyklen ganz oder teilweise gesättigt sein. Bevorzugte Beispiele für Aryl sind Phenyl, Naphthyl oder Anthracyl, insbesondere Phenyl.

Nachfolgend wird die vorliegende Erfindung weiter präzisiert.

Gegenstand der vorliegenden Erfindung ist zunächst ein 2,6-bis-(Aminomethyl)piperidin-Derivat (2,6-BAMP-Derivat) gemäß der allgemeinen Formel (I), 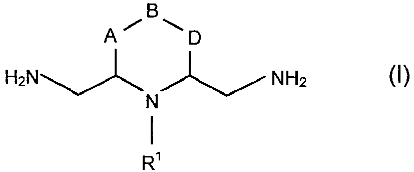 wobei:
- A, B und D: sind unabhängig voneinander CH₂, CHR² oder CR²R³;
- R¹: ist unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
- R² und R³: sind unabhängig voneinander unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
- R⁴ und R⁵: sind unabhängig voneinander Wasserstoff oder unsubstituiertes C₁-C₄-Alkyl;
- R⁶: ist unsubstituiertes C₁-C₁₀-Alkyl.

Vorzugsweise ist das 2,6-BAMP-Derivat dadurch gekennzeichnet, dass das 2,6-BAMP-Derivat gemäß der allgemeinen Formel (la) definiert ist, 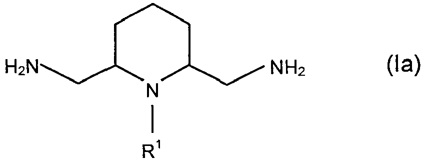 wobei:
- R¹: ist unsubstituiertes oder zumindest monosubstituiertes C₁-C₁₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -NR⁴R⁵ oder Halogen,
und Aryl² kann wiederum mit C₁-C₄-Alkyl, -OR⁴, -NR⁴R⁵ oder Halogen zumindest monosubstituiert sein;
- R⁴ und R⁵: sind unabhängig voneinander Wasserstoff oder unsubstituiertes C₁-C₄-Alkyl.

Mehr bevorzugt ist das 2,6-BAMP-Derivat der allgemeinen Formel (I) und noch mehr bevorzugt ist das 2,6-BAMP-Derivat der allgemeinen Formal (la) wie folgt definiert:
- R¹: ist unsubstituiertes oder zumindest monosubstituiertes C₁-C₄-Alkyl und der Substituent ist -NR⁴R⁵;
- R⁴ und R⁵: sind unabhängig voneinander Wasserstoff oder unsubstituiertes C₁-C₄-Alkyl;
insbesondere ist R¹ Wasserstoff. Im Fall der allgemeinen Formel (la) bedeutet dies bei dieser (mehr bevorzugten) Ausführungsform, dass es sich dabei um 2,6-BAMP als solchen handelt.

In der vorstehenden Definition der allgemeinen Formel (I) oder (la) sind Aryl oder Aryl² vorzugsweise Phenyl, Naphthyl oder Anthracyl, insbesondere Phenyl.

Ein weiterer Gegenstand der vorliegenden Erfindung ist ein Verfahren zur Herstellung eines 2,6-bis-(Aminomethyl)piperidinderivats (2,6-BAMP-Derivat) gemäß der allgemeinen Formel (I), vorzugsweise gemäß der allgemeinen Formel (la)..

Wobei in dem Verfahren zur Herstellung des 2,6-bis-(Aminomethyl)piperidinderivats (2,6-BAMP-Derivat) gemäß der allgemeinen Formel (I), in der allgemeinen Formel (I),
- A, B und D: sind unabhängig voneinander CH₂, CHR² oder CR²R³;
- R¹: ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
- R² und R³: sind unabhängig voneinander unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
- R⁴ und R⁵: sind unabhängig voneinander Wasserstoff oder unsubstituiertes C₁-C₄-Alkyl;
- R⁶: ist unsubstituiertes C₁-C₁₀-Alkyl.

Im übrigen gelten die vorstehenden Definitionen.

Wobei in dem Verfahren zur Herstellung des 2,6-bis-(Aminomethyl)piperidinderivats (2,6-BAMP-Derivat) gemäß der allgemeinen Formel (la), in der allgemeinen Formel (Ia),
- R¹: ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
- R⁴ und R⁵: sind unabhängig voneinander Wasserstoff oder unsubstituiertes C₁-C₄-Alkyl;

Im übrigen gelten die vorstehenden Definitionen.

Im erfindungsgemäßen Verfahren kann gezielt ein einziges 2,6-BAMP-Derivat oder auch ein Gemisch aus zwei oder mehreren solcher Derivate, die unter die vorstehend beschriebenen Definitionen fallen, hergestellt werden. Im erfindungsgemäßen Verfahren wird das 2,6-BAMP-Derivat aus dem entsprechenden 2,6-Dicyanopiperidin-Derivat (2,6-DCP-Derivat) gemäß der allgemeinen Formeln (II) oder (IIa) durch Hydrierung in Gegenwart eines Katalysators erhalten.

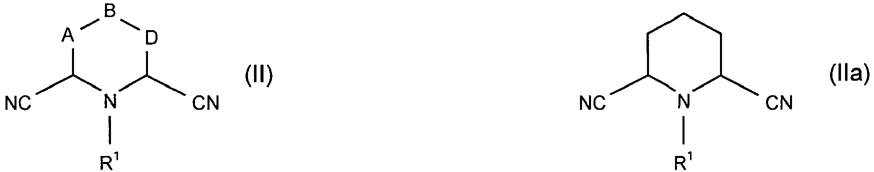

Wobei in dem Verfahren zur Herstellung des 2,6-bis-(Aminomethyl)piperidinderivats (2,6-BAMP-Derivat) in der allgemeinen Formel (II)
- A, B und D: sind unabhängig voneinander CH₂, CHR² oder CR²R³;
- R¹: ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
- R² und R³: sind unabhängig voneinander unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
- R⁴ und R⁵: sind unabhängig voneinander Wasserstoff oder unsubstituiertes C₁-C₄-Alkyl;
- R⁶: ist unsubstituiertes C₁-C₁₀-Alkyl.

Im übrigen gelten die vorstehenden Definitionen.

Wobei in dem Verfahren zur Herstellung des 2,6-bis-(Aminomethyl)piperidinderivats (2,6-BAMP-Derivat) in der allgemeinen Formel (IIa),
- R¹: ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen, und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
- R⁴ und R⁵: sind unabhängig voneinander Wasserstoff oder unsubstituiertes C₁-C₄-Alkyl;

Im übrigen gelten die vorstehenden Definitionen.

Die 2,6-DCP-Derivate gemäß der allgemeinen Formel (II) oder (IIa) als solche sind, wie vorstehend bereits ausgeführt, bekannt. Entsprechende Verbindungen sowie die zugehörigen Herstellungsverfahren sind beispielsweise in GB-A 915 227 oder CHEM. REV. 1983 (4), Seiten 379-423, beschrieben. Der Vollständigkeit halber wird nochmals darauf hingewiesen, dass in der Formel (II) sämtliche Substituenten (R¹ bis R⁶) sowie Variablen (A, B, D) immer die gleiche Bedeutung haben wie vorstehend für das entsprechende 2,6-BAMP-Derivat gemäß der allgemeinen Formel (I) beschrieben. Sinngemäßes gilt auch für die Definitionen/das Verhältnis des 2,6-DCP-Derivats gemäß der allgemeinen Formel (IIa) zum vorstehend beschriebenen 2,6-BAMP-Derivat gemäß der allgemeinen Formel (la) sowie die weiteren im vorstehenden Text aufgeführten bevorzugten Ausführungsformen bezüglich der erfindungsgemäßen 2,6-BAMP-Derivate.

In einer bevorzugten Ausführungsform wird das zur Hydrierung eingesetzte 2,6-DCP-Derivat durch Umsetzung von i) Glutaraldehyd oder einem Glutaraldehyd-Derivat mit ii) Cyanowasserstoff (HCN) und iii) einem entsprechenden Amin der Formel R¹NH₂ hergestellt.

Der Vollständigkeit halber wird darauf hingewiesen, dass bei dieser Ausführungsform im Falle der Verwendung von Glutaraldehyd solche 2,6-DCP-Derivate gemäß der allgemeinen Formel (II) hergestellt werden, bei denen die Variablen A, B und D allesamt CH₂ sind. Sinngemäß werden Glutaraldehyd-Derivate entsprechend eingesetzt, um in der allgemeinen Formel (II) für 2,6-DCP-Derivate die weiteren Definitionen (CHR² und CR²R³) für die Variablen A, B und D zu verwirklichen.

Das bei dieser Ausführungsform eingesetzte Amin mit der Formel R¹NH₂ ist dem Fachmann ebenfalls bekannt. Die Definition des Restes R¹ entspricht dabei der Definition des Restes R¹ in den 2,6-DCP-Derivaten gemäß der allgemeinen Formel (II) oder (IIa).

Die Hydrierung unter Erhalt der erfindungsgemäßen 2,6-BAMP-Derivate erfolgt im Allgemeinen durch Umsetzung der 2,6-DCP-Derivate mit Wasserstoff in Gegenwart eines Hydrierkatalysators.

Bei der Hydrierung zum 2,6-BAMP-Derivat werden im Allgemeinen mindestens vier Mole Wasserstoff pro Mol 2,6-DCP-Derivat benötigt.

Die Temperaturen, bei denen die Hydrierung durchgeführt wird, liegen in einem Bereich von 60 bis 180 °C, bevorzugt von 80 bis 140 °C, insbesondere bei 90 bis 130 °C.

Der bei der Hydrierung herrschende Druck liegt im Allgemeinen bei 40 bis 300 bar, bevorzugt bei 40 bis 240 bar, besonders bevorzugt bei 80 bis 200 bar.

In einer bevorzugten Ausführungsform wird das 2,6-DCP-Derivat gemäß der Formel (II) mit einer Rate der Hydrierung zugeführt, die nicht größer ist als die Rate, mit der das 2,6-DCP-Derivat mit Wasserstoff bei der Hydrierung reagiert.

Die Zuführrate ist bevorzugt so einzustellen, dass quasi Vollumsatz erreicht wird. Dieses wird durch Temperatur, Druck, Art des 2,6-DCP-Derivats, Menge und Art des Katalysators, des Reaktionsmediums, Durchmischungsgüte des Reaktorinhalts, Verweilzeit etc. beeinflusst.

Das erfindungsgemäße Verfahren wird in Gegenwart eines Katalysators durchgeführt. Als Katalysatoren können prinzipiell alle dem Fachmann für eine Nitrilhydrierung bekannten Katalysatoren eingesetzt werden. Als Katalysatoren zur Hydrierung der beiden Nitrilfunktionen zum 2,6-BAMP-Derivat können somit beispielsweise Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten.

Darin eingeschlossen sind so genannte oxidische Katalysatoren, die die ein oder mehrere aktive Spezies in Form ihrer sauerstoffhaltigen Verbindungen enthalten und so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten.

In einer besonders bevorzugten Ausführungsform werden bei Hydrierung von der 2,6-DCP-Derivate Raney-Katalysatoren eingesetzt, bevorzugt Raney-Kobalt- oder Raney-Nickel-Katalysatoren und besonders bevorzugt ein Raney-Nickel-Katalysator, der als Promotor mindestens eines der Elemente Ni, Cr oder Fe enthält. Der Raney-Kobalt-Katalysator ist also mit mindestens einem dieser Elemente dotiert.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoffenthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können. Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O3 bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 1 1 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als "Metallschwamm" aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach zum Beispiel mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw. Die Aktivierung der Katalysatoren durch Auslaugen der löslichen Komponente (typscherweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1 - 30 Gew.-% Al, besonders 2-12 Gew.-% Al, ganz besonders 3-6 Gew.-% Al, 0-10 Gew.-% Cr, bei-sonders 0,1-7 Gew.-% Cr, ganz besonders 0,5-5 Gew.-% Cr, insbesondere 1,5 - 3,5 Gew.-% Cr, 0-10 Gew.-% Fe, besonders 0,1-3 Gew.-% Fe, ganz besonders 0,2-1 Gew.-% Fe, und/oder 0-10 Gew.-% Ni, besonders 0,1-7 Gew.-% Ni, ganz besonders 0,5-5 Gew. % Ni, insbesondere 1-4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf: Al: 2-6 Gew.-%, Co: ≥ 86 Gew.-%, Fe: 0-1 Gew.-%, Ni: 1-4 Gew.-%, Cr: 1,5-3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch 1-30 Gew.-% Al, besonders 2-20 Gew.-% Al, ganz besonders 5-14 Gew.-% Al, 0-10 Gew.-% Cr, besonders 0,1-7 Gew.-% Cr, ganz besonders 1-4 Gew.-% Cr, und/oder 0-10 Gew.-% Fe, besonders 0,1-7 Gew.-% Fe, ganz besonders 1 - 4 Gew.-% Fe, wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden. Dieser Katalysator weist folgende Zusammensetzung auf:
Al: <14 Gew.-%, Ni: ≥ 80 Gew.-%, Fe: 1-4 Gew.%, Cr: 1-4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Das erfindungsgemäße Verfahren kann in Gegenwart eines Lösungsmittels durchgeführt werden. Als Lösungsmittel eignen sich prinzipiell alle dem Fachmann bekannten Lösungsmittel, wobei sich die Lösungsmittel vorzugsweise gegenüber den 2,6-DCP-Derivaten inert verhalten.

Mögliche Lösungsmittel sind organische Lösungsmittel, beispielsweise Amide, wie N-Methylpyrrolidon (NMP) und Dimethylformamid (DMF), aromatische und aliphatische Kohlenwasserstoffe, wie Toluol, Alkohole wie Methanol, Ethanol, Propanol, Isopropanol, Butanol, Isobutanol, sekundäres Butanol und tertiäres Butanol, Amine, wie EDA bzw. Ethylamine und Ammoniak, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF).

Vorzugsweise ist das Lösungsmittel ein Amid, ein aromatischer Kohlenwasserstoff, ein Alkohol, ein Amin, ein Ester oder ein Ether. Mehr bevorzugt werden im erfindungsgemäßen Verfahren Ether verwendet, noch mehr bevorzugt zyklische Ether und besonders bevorzugt Tetrahydrofuran.

Das Lösungsmittel wird normalerweise im Gewichtsverhältnis zu dem eingesetzten 2,6-DCP-Derivat von 0,1:1 bis 15:1 eingesetzt. Die Konzentration des 2,6-DCP-Derivats in der Lösung, in der die Hydrierung durchgeführt wird, sollte so gewählt werden, dass eine geeignete Zuführrate bzw. Verweilzeit eingestellt werden kann. Es ist bevorzugt, das 2,6-DCP-Derivat zu 10 bis 50 Gew.-% mit dem Lösungsmittel zu vermischen. Bezogen auf die besonders bevorzugten Lösungsmittel Tetrahydrofuran ist es beispielsweise vorteilhaft, das 2,6-DCP-Derivat zu 20 bis 40 Gew.-% bezogen auf das Lösungsmittel einzusetzen.

Die Umsetzung der 2,6-DCP-Derivate mit Wasserstoff in Gegenwart von Katalysatoren kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des 2,6-DCP-Derivats und des Katalysators mit dem Wasserstoff unter Druck möglich ist.

Die Hydrierung in Suspensionsfahrweise kann in einem Rührreaktor, Strahlschlaufenreaktor, Strahldüsenreaktor, Blasensäulenreaktor bzw. in einer Kaskade derartiger gleicher oder verschiedener Reaktoren durchgeführt werden.

Die Hydrierung an einem Festbettkatalysator findet bevorzugt in einem oder mehreren Rohrreaktoren aber auch Rohrbündelreaktoren statt.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmäntel oder außen liegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes.

Insbesondere kann der Kreislaufstrom mittels eines externen Wärmeüberträgers auf einfache und kostengünstige Weise gekühlt und somit die Reaktionswärme abgeführt werden.

Der Reaktor kann auch adiabat betrieben werden. Bei adiabatem Betrieb des Reaktors kann der Temperaturanstieg im Reaktionsgemisch durch Abkühlung der Zuläufe oder durch Zufuhr von "kaltem" organischem Lösungsmittel begrenzt werden.

Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Der Katalysator kann in einem Festbett angeordnet (Festbettfahrweise) sein oder im Reaktionsgemisch suspendiert (Suspensionsfahrweise) sein.

In einer besonders bevorzugten Ausführungsform ist der Katalysator im zu hydrierenden Reaktionsgemisch suspendiert.

Die Absetzgeschwindigkeit des Hydrierkatalysators in dem gewählten Lösungsmittel sollte niedrig sein, damit der Katalysator gut in Suspension gehalten werden kann.

Die Partikelgröße der eingesetzten Katalysatoren beträgt bei der Suspensionsfahrweise daher bevorzugt zwischen 0,1 und 500 µm, insbesondere 1 und 100 µm.

Wird die Hydrierung der 2,6-DCP-Derivate in Suspensionsfahrweise kontinuierlich durchgeführt, so werden die 2,6-DCP-Derivate bevorzugt kontinuierlich dem Reaktor zugeführt und aus dem Reaktor kontinuierlich ein Strom entfernt, der die Hydrierungsprodukte (2,6-BAMP-Derivate) enthält.

Bei der diskontinuierlichen Suspensionsfahrweise werden die 2,6-DCP-Derivate gegebenenfalls zusammen mit organischem Lösungsmittel vorgelegt.

Die Menge an Katalysator bei der diskontinuierlichen Suspensionsfahrweise beträgt bevorzugt 1 bis 60 Gew.-%, besonders bevorzugt 5 bis 40 Gew.-%, und ganz besonders bevorzugt 20 bis 30 Gew.-%, bezogen auf das gesamte Reaktionsgemisch.

Die Verweilzeit im Reaktor beträgt bei diskontinuierlicher Suspensions-Fahrweise bevorzugt 0,1 bis 6 Stunden, besonders bevorzugt 0,5 bis 2 Stunden.

Die Verweilzeit im Reaktor beträgt bei kontinuierlicher Suspensionsfahrweise bevorzugt 0,1 bis 6 Stunden, besonders bevorzugt 0,5 bis 2 Stunden.

Die Katalysatorbelastung beträgt bei der kontinuierlichen Suspensionsfahrweise bzw. beim Semi-Batch-Verfahren 0,1 bis 5 kg, bevorzugt 0,1 bis 2 kg, besonders bevorzugt 0,1 bis 1 kg an 2,6-DCP-Derivat pro kg Katalysator und Stunde.

Wird die Reaktion in Suspensionsfahrweise in einem Rührreaktor durchgeführt, so liegt der Leistungseintrag über den Rührer bevorzugt bei 0,1 bis 100 KW pro m³.

Gebrauchter Katalysator kann durch Filtration, Zentrifugieren oder Querstromfiltration abgetrennt werden. Dabei kann es notwendig sein, Verluste an ursprünglicher Katalysatormenge durch Abrieb und/oder Desaktivierung durch Zugabe von frischem Katalysator auszugleichen.

Im Anschluss an die Hydrierung kann der Austrag aus der Hydrierung gegebenenfalls weiter aufgereinigt werden. Der Katalysator kann nach dem Fachmann bekannten Methoden abgetrennt werden. In der Regel wird nach Abtrennung des Katalysators der während der Hydrierung vorhandene Wasserstoff abgetrennt.

Die Abtrennung von Wasserstoff erfolgt bevorzugt durch Absenkung des Drucks, bei dem die Hydrierung durchgeführt wurde, auf einen Wert, bei dem Wasserstoff gasförmig ist, die anderen Komponenten im Reaktionsaustrag aber in der Flüssigphase vorliegen. Vorzugsweise wird der Reaktionsaustrag von einem Hydrierdruck von bevorzugt 60 bis 325 bar, besonders bevorzugt 100 bis 280 bar, und ganz besonders bevorzugt 170 bis 240 bar auf einen Druck von 5 bis 50 bar in einen Behälter entspannt. Am Kopf des Behälters werden Wasserstoff, gegebenenfalls Ammoniak, sowie gegebenenfalls geringe Menge an verdampften Leichtsiedern oder Lösungsmitteln, wie THF, erhalten. Wasserstoff und gegebenenfalls Ammoniak können in die Hydrierung der 2,6-DCP-Derivate zurückgeführt werden. Beispielsweise kann THF auskondensiert und zurückgewonnen werden.

Im Reaktionsaustrag gegebenenfalls vorhandene organische Lösungsmittel werden im Allgemeinen ebenfalls destillativ abgetrennt. Insbesondere können die erfindungsgemäßen 2,6-BAMP-Derivate nach dem Fachmann bekannten Methoden aus dem Reaktionsprodukt isoliert werden.

Die vorliegende Erfindung betrifft zudem die Verwendung von 2,6-BAMP-Derivaten gemäß der allgemeinen Formel (I), vorzugsweise gemäß der allgemeinen Formel (Ia) wie für das Verfahren zur Herstellung des bis-(Aminomethyl)piperidinderivats (2,6-BAMP-Derivat) definiert, als Härter für Epoxyharze, als Zwischenprodukt bei der Herstellung von Diisocyanaten, als Starter bei der Herstellung von Polyetherolen und/oder als Monomer für die Polyamidherstellung.

2,6-BAMP-Derivate gemäß der allgemeinen Formeln (I), vorzugsweise gemäß der allgemeinen Formel (Ia) wie für das Verfahren zur Herstellung des bis-(Aminomethyl)piperidinderivats (2,6-BAMP-Derivat) definiert, stellen alternative Härter für Epoxyharze dar, die neue Möglichkeiten bei der Formulierung und Verarbeitung von Epoxyharzen ermöglichen und zur Regulierung des Eigenschaftsspektrums von Epoxyharzen eingesetzt werden können.

2,6-BAMP-Derivate gemäß der allgemeinen Formel (I), vorzugsweise gemäß der allgemeinen Formel (la) wie für das Verfahren zur Herstellung des bis-(Aminomethyl)piperidinderivats (2,6-BAMP-Derivat) definiert können auch als Zwischenprodukt bei der Herstellung der entsprechenden Diisocyanate der nachstehend aufgeführten allgemeinen Formel (III) bzw. Formel (IIIa) verwendet werden. In den allgemeinen Formeln (III) und (IIIa) haben die jeweiligen Reste (wie R¹) und Variablen (wie A) die gleiche Bedeutung wie vorstehend für die allgemeinen Formeln (I) und (la) für das Verfahren zur Herstellung des bis-(Aminomethyl)piperidinderivats (2,6-BAMP-Derivat) definiert.

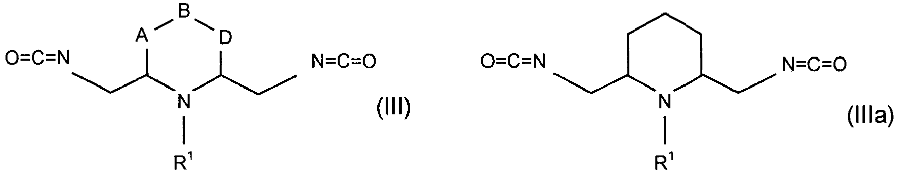

Diese Diisocyanate sind für die Herstellung von lichtbeständigen Polyurethanen, beispielsweise als Lack oder Beschichtung, geeignet und bieten aufgrund ihrer Struktur neue Formulierungsmöglichkeiten und damit Zugang zu neuen, interessanten Eigenschaftsprofilen. Diese Diisocyanate sind beispielsweise durch Umsetzung von 2,6-BAMP-Derivaten mit Phosgen erhältlich.

2,6-BAMP-Derivate gemäß der allgemeinen Formel (I), vorzugsweise gemäß der allgemeinen Formel (la) wie für das Verfahren zur Herstellung des bis-(Aminomethyl)piperidinderivats (2,6-BAMP-Derivat) definiert, können auch als Starter bei der Herstellung von Polyetherolen verwendet werden. 2,6-BAMP-Derivate sind CHacide Verbindungen, die mit einer Base deprotoniert werden können und an die nachfolgenden Alkylenoxide, wie Ethylenoxid, Propylenoxid und/oder Butylenoxid, addiert werden können. Alkoxilierte Diamine können beispielsweise als Katalysatoren in der PUR-Herstellung eingesetzt werden.

2,6-BAMP-Derivate gemäß der allgemeinen Formel (I), vorzugsweise gemäß der allgemeinen Formel (la) wie für das Verfahren zur Herstellung des bis-(Aminomethyl)piperidinderivats (2,6-BAMP-Derivat) definiert können als Monomer bei der Herstellung von Polyamiden eingesetzt werden. So können 2,6-BAMP-Derivate beispielsweise mit Dicarbonsäuren, wie beispielsweise Bernsteinsäure, Adipinsäure, Terephthalsäure und/oder Phthalsäure, zu Polymeren umgesetzt werden.

Ein weiterer Gegenstand der vorliegenden Erfindung ist somit auch ein Diisocyanat gemäß der allgemeinen Formel (III), 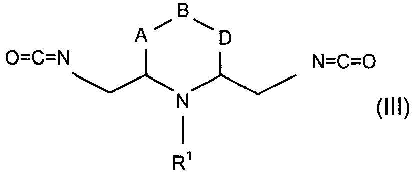 wobei:
- R¹: ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl, wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
- R² und R³: sind unabhängig voneinander unsubstituiertes oder zumindest monosubstituiertes C₁-C₁₀-Alkyl oder unsubstituiertes oder zumindest monosubstituiertes Aryl, wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
- R⁴ und R⁵: sind unabhängig voneinander Wasserstoff oder unsubstituiertes C₁-C₄-Alkyl;
- R⁶: ist unsubstituiertes C₁-C₁₀-Alkyl.

Vorzugsweise ist das Diisocyanat gemäß der allgemeinen Formel (III) durch die allgemeine Formel (IIIa) definiert, 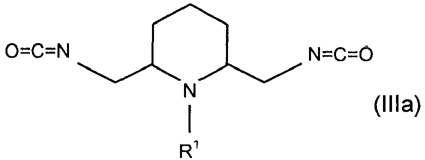 wobei:
- R¹: ist Wasserstoff, unsubstituiertes oder zumindest monosubstituiertes C₁-C₁₀-Alkyl, unsubstituiertes oder zumindest monosubstituiertes C₂-C₁₀-Alkenyl oder unsubstituiertes oder zumindest monosubstituiertes Aryl, wobei die Substituenten ausgewählt sind aus der Gruppe bestehend aus C₁-C₄-Alkyl, Aryl², -OR⁴, -NR⁴R⁵ oder Halogen, und Aryl² kann wiederum mit C₁-C₄-Alkyl, -OR⁴, -NR⁴R⁵ oder Halogen zumindest monosubstituiert sein;
- R⁴ und R⁵: sind unabhängig voneinander Wasserstoff oder unsubstituiertes C₁-C₄-Alkyl.

Noch mehr bevorzugt sind in den erfindungsgemäßen Diisocyanaten gemäß der allgemeinen Formel (III) oder allgemeinen Formel (IIIa) folgende Definitionen vorgesehen:
- R¹: ist Wasserstoff, unsubstituiertes oder zumindest monosubstituiertes C₁-C₄-Alkyl und der Substituent ist -NR⁴R⁵;
- R⁴ und R⁵: sind unabhängig voneinander Wasserstoff oder unsubstituiertes C₁-C₄-Alkyl;
vorzugsweise ist R¹ Wasserstoff.

Ein weiterer Gegenstand der vorliegenden Erfindung ist somit auch ein Verfahren zur Herstellung eines Diisocyanats gemäß den vorstehend beschriebenen Definitionen. Verfahren zur Herstellung von Diisocyanaten aus den entsprechenden Diaminen, beispielsweise durch Umsetzung mit Phosgen, sind dem Fachmann bekannt, so dass der Fachmann diese Verfahren entsprechend anwenden kann.

Erfindungsgemäß werden die Diisocyanate vorzugsweise hergestellt, indem ein entsprechendes 2,6-BAMP-Derivat gemäß der allgemeinen Formel (I), vorzugsweise gemäß der allgemeinen Formel (la) wie für das Verfahren zur Herstellung des bis-(Aminomethyl)piperidinderivats (2,6-BAMP-Derivat) definiert, mit Phosgen umgesetzt wird. Vorzugsweise wird dabei ein 2,6-BAMP-Derivat eingesetzt, gegebenenfalls können auch Gemische von zwei oder mehreren solcher Derivate eingesetzt werden.

Nachfolgend wird die Erfindung anhand von Beispielen verdeutlicht.

### Beispiel 1

### Herstellung von 2,6-Dicyanopiperidin

62 g Wasser werden vorgelegt und auf 5 °C abgekühlt. Anschließend werden parallel innerhalb von 60 Minuten 175,0 g Ammoniakwasser 25 gew.-%ig, 250,0 g einer 50 gew.-%igen wässrigen Glutaraldehyd-Lösung und 68,3 g Blausäure zudosiert, wobei der Zulauf von Blausäure ca. 10 Minuten früher beginnt. Die Zuläufe erfolgen so, dass eine Temperatur von 10 °C nicht überschritten wird. Nach einer Nachreaktion von 12 Stunden bei maximal 10 °C wird der entstandene Niederschlag abgesaugt. Es werden 156 g eines wasserfeuchten, weißen Feststoffs erhalten. Zur Entfernung des Wassers wird dieser in 700 ml Tetrahydrofuran aufgenommen, mit Natriumsulfat getrocknet und filtriert. Nach Einengen des Filtrats am Rotationsverdampfer werden 109 g eines weißen Feststoffs erhalten, welcher mittels 13C-NMR als 2,6-Dicyanopiperidin charakterisiert wird.

### Beispiel 2

### Hydrierung (Semi-Batch)

In einem 270 ml Autoklaven mit Strombrechern und Scheibenrührer wurden 5 g (trocken) Raney-Kobalt und 40 g THF vorgelegt. Der Autoklav wird auf 120 °C aufgeheizt und Wasserstoff bis zu einem Gesamtdruck von 100 bar aufgepresst. Innerhalb von 5,5 Stunden wird eine Mischung aus 8 g 2,6-Dicyanopiperidin (hergestellt nach Beispiel 1) in 72 g THF zudosiert. Die Reaktionsmischung wird weitere 60 Minuten unter Reaktionsbedingungen gerührt. Der Hydrieraustrag enthält 90 % 2,6-bis-(Aminomethyl)-piperidin (GC Flächen-%). Der Rest sind unbekannte Nebenkomponenten.

### GC-Methode: DB1 Säule, 30 m, 0,32 mm, 1 µm; 80 °C Anfangstemperatur, Temperaturrampe 10 °C/min auf 280 °C

### Beispiel 3

### Hydrierung (kontinuierlich)

In einem 270 ml Autoklaven mit Stromstörern und Scheibenrührer wurden 5 g (trocken) Raney-Kobalt in 40 g THF vorgelegt. Es wird kontinuierlich 15 Nl/h Wasserstoff zugefahren. 38 g einer Mischung aus 10 g 2,6-Dicyanopiperidin (hergestellt nach Beispiel 1) in 90 g THF werden kontinuierlich pro Stunde bei 200 bar zugepumpt. Die Temperatur im Reaktor beträgt 120 °C. Der Katalysator wird durch kontinuierliche Filtration über eine Sintermetallfritte mit einem Porendurchmesser von 500 nm vom Reaktoraustrag getrennt. Der Austrag wird über ein Regelventil entspannt. In einem nachgeschalteten Phasenscheider wird anschließend Wasserstoff abgetrennt. Insgesamt werden 114 g 2,6-Dicyanopiperidin eingesetzt.

Das rohe Reaktionsgemisch wird zunächst im Rotationsverdampfer eingeengt und über eine Vigreux-Kolonne bei < 0,5 mbar destilliert. Bei 75 °C geht das Produkt über Kopf. Es werden 73 g 2,6-bis-(Aminomethyl)-piperidin mit einer Reinheit von 90-95 % erhalten. Die Ausbeute in der Hydrierstufe beträgt 75 % unter Einrechnung des Produktgehaltes in dem Rohaustrag und 60,8 % an Produkt nach der Destillation.

Das Produkt wurde durch GC-MS und NMR charakterisiert.
13C-NMR (125 MHz, THF): 60.31, 49.42, 31.4, 25.62
GC-MS: DB1 Säule, 30 m, 0,32 mm, 1 µm; 80 °C Anfangstemperatur, Temperaturrampe 10 °C/min auf 280 °C - Retentionszeit 9,06 min (93,3 FI.-%).

## Patentansprüche

1. 2,6-bis-(Aminomethyl)piperidin-Derivat (2,6-BAMP-Derivat) gemäß der allgemeinen Formel (I), 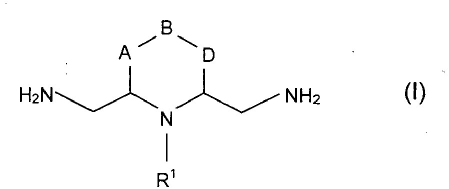 wobei:
A, B und D sind unabhängig voneinander CH₂, CHR² oder CR²R³;
R¹ ist unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl, wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen, und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
R² und R³ sind unabhängig voneinander unsubstituiertes oder zumindest monosubstituiertes C₁-C₁₀-Alkyl oder unsubstituiertes oder zumindest monosubstituiertes Aryl, wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
R⁴ und R⁵ sind unabhängig voneinander Wasserstoff oder unsubstituiertes C₁-C₄-Alkyl;
R⁶ ist unsubstituiertes C₁-C₁₀-Alkyl.

2. 2,6-BAMP-Derivat gemäß Anspruch 1, **dadurch gekennzeichnet, dass** das 2,6-BAMP-Derivat gemäß der allgemeinen Formel (la) definiert ist, 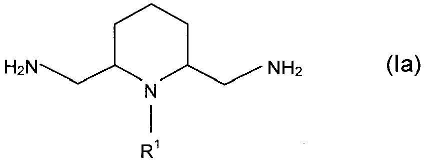 wobei:
R¹ ist unsubstituiertes oder zumindest monosubstituiertes C₁-C₁₀-Alkyl, unsubstituiertes oder zumindest monosubstituiertes C₂-C₁₀-Alkenyl oder unsubstituiertes oder zumindest monosubstituiertes Aryl, wobei die Substituenten ausgewählt sind aus der Gruppe bestehend aus C₁-C₄-Alkyl, Aryl², -OR⁴, -NR⁴R⁵ oder Halogen, und Aryl² kann wiederum mit C₁-C₄-Alkyl, -OR⁴, -NR⁴R⁵ oder Halogen zumindest monosubstituiert sein;
R⁴ und R⁵ sind unabhängig voneinander Wasserstoff oder unsubstituiertes C₁-C₄-Alkyl.

3. 2,6-BAMP-Derivat gemäß Anspruch 1 oder 2, wobei:
R¹ ist unsubstituiertes oder zumindest monosubstituiertes C₁-C₄-Alkyl und der Substituent ist -NR⁴R⁵;
R⁴ und R⁵ sind unabhängig voneinander Wasserstoff oder unsubstituiertes C₁-C₄-Alkyl.

4. Verfahren zur Herstellung eines 2,6-bis-(Aminomethyl)piperidin-Derivats (2,6-BAMP-Derivat) gemäß der allgemeinen Formel (I), 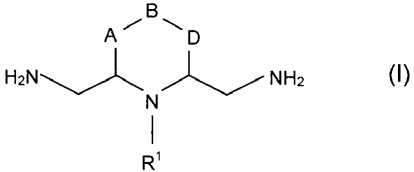 **dadurch gekennzeichnet, dass** das 2,6-BAMP-Derivat aus dem entsprechenden 2,6-Dicyanopiperidin-Derivat (2,6-DCP-Derivat) gemäß der allgemeinen Formeln (II) oder (IIa) 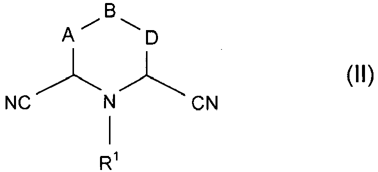 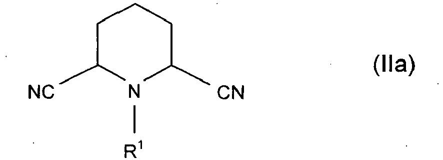 durch Hydrierung in Gegenwart eines Katalysators erhalten wird, wobei:
A, B und D sind unabhängig voneinander CH₂, CHR² oder CR²R³;
R¹ ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
R² und R³ sind unabhängig voneinander unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
R⁴ und R⁵ sind unabhängig voneinander Wasserstoff oder unsubstituiertes C₁-C₄-Alkyl;
R⁶ ist unsubstituiertes C₁-C₁₀-Alkyl.

5. Verfahren gemäß Anspruch 4, **dadurch gekennzeichnet, dass** als Katalysator ein Raney-Katalysator eingesetzt wird, bevorzugt ein Raney-Nickel- oder ein Raney-Kobalt-Katalysator, insbesondere ein Raney-Kobalt-Katalysator, der als Promotor mindestens eines der Elemente Fe, Ni oder Cr enthält.

6. Verfahren gemäß Anspruch 4 oder 5, **dadurch gekennzeichnet, dass** die Hydrierung in einem Lösungsmittel durchgeführt wird, vorzugsweise ist das Lösungsmittel ein Amid, ein aromatischer Kohlenwasserstoff, ein Alkohol, ein Amin, ein Ester oder ein Ether, insbesondere ist das Lösungsmittel Tetrahydrofuran.

7. Verfahren gemäß einem der Ansprüche 4 bis 6, **dadurch gekennzeichnet, dass** die Hydrierung bei einem Druck von 80 bis 200 bar und/oder bei einer Temperatur von 90 bis 130 °C durchgeführt wird.

8. Verfahren gemäß einem der Ansprüche 4 bis 7, **dadurch gekennzeichnet, dass** das zur Hydrierung eingesetzte 2,6-DCP-Derivat durch Umsetzung von i) Glutaraldehyd oder einem Glutaraldehyd-Derivat mit ii) Cyanowasserstoff (HCN) und iii) einem entsprechenden Amin der Formel R¹NH₂ hergestellt wird.

9. Verwendung eines 2,6-BAMP-Derivats gemäß der Definition der allgemeinen Formel (I) in Anspruch 4 als Härter für Epoxyharze, als Zwischenprodukt bei der Herstellung von Diisocyanaten, als Starter bei der Herstellung von Polyetherolen und/oder als Monomer für die Polyamidherstellung.

10. Diisocyanat gemäß der allgemeinen Formel (III), 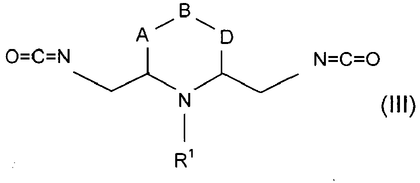 wobei:
A, B und D sind unabhängig voneinander CH₂, CHR² oder CR²R³;
R¹ ist Wasserstoff, unsubstituiertes oder zumindest monosubstituiertes C₁-C₃₀-Alkyl, unsubstituiertes oder zumindest monosubstituiertes C₂-C₁₀-Alkenyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -C(O)R⁶, -C(O)OR⁴, -O-C(O)R⁶, -NR⁴R⁵ oder Halogen,
und Aryl² kann wiederum mit C₁-C₄-Alkyl, -OR⁴, -C(O)R⁶, -C(O)OR⁴, -O-C(O)R⁶, -NR⁴R⁵ oder Halogen zumindest monosubstituiert sein;
R² und R³ sind unabhängig voneinander unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, -OR⁴, -C(O)R⁶, -C(O)OR⁴, -O-C(O)R⁶, -NR⁴R⁵ oder Halogen;
R⁴ und R⁵ sind unabhängig voneinander Wasserstoff oder unsubstituiertes C₁-C₄-Alkyl;
R⁶ ist unsubstituiertes C₁-C₁₀-Alkyl.

11. Diisocyanat gemäß Anspruch 10, **dadurch gekennzeichnet, dass** das Diisocyanat gemäß der allgemeinen Formel (IIIa) definiert ist, 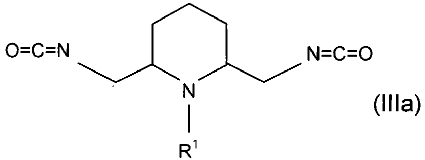 wobei:
R¹ ist Wasserstoff, unsubstituiertes oder zumindest monosubstituiertes C₁-C₁₀-Alkyl oder unsubstituiertes oder zumindest monosubstituiertes Aryl,
wobei die Substituenten ausgewählt sind aus der Gruppe bestehend aus C₁-C₄-Alkyl, Aryl², -OR⁴, -NR⁴R⁵ oder Halogen,
und Aryl² kann wiederum mit C₁-C₄-Alkyl, -OR⁴, -NR⁴R⁵ oder Halogen zumindest monosubstituiert sein;
R⁴ und R⁵ sind unabhängig voneinander Wasserstoff oder unsubstituiertes C₁-C₄-Alkyl.

12. Verfahren zur Herstellung eines Diisocyanats gemäß Anspruch 10 oder 11, **dadurch gekennzeichnet, dass** ein 2,6-BAMP-Derivat gemäß der Definition der allgemeinen Formel (II) in Anspruch 4 mit Phosgen umgesetzt wird.

## Claims

1. A 2,6-bis(aminomethyl)piperidine derivative (2,6-BAMP derivative) of the general formula (I), 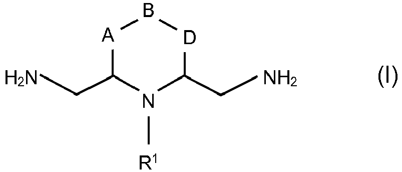 where:
A, B and D are each, independently of one another, CH₂, CHR² or CR²R³;
R¹ is unsubstituted or at least monosubstituted C₁-C₃₀-alkyl, unsubstituted or at least monosubstituted C₂-C₁₀-alkenyl or unsubstituted or at least monosubstituted aryl,
where the substituents are selected from the group consisting of C₁-C₄-alkyl, aryl², -OR⁴, -C(O)R⁶, -C(O)OR⁴, -O-C(O)R⁶, -NR⁴R⁵ or halogen,
and aryl² can in turn be at least monosubstituted by C₁-C₄-alkyl, -OR⁴, -C(O)R⁶, -C(O)OR⁴, -O-C(O)R⁶, -NR⁴R⁵ or halogen;
R² and R³ are each, independently of one another, unsubstituted or at least monosubstituted C₁-C₁₀-alkyl or unsubstituted or at least monosubstituted aryl,
where the substituents are selected from the group consisting of C₁-C₄-alkyl, -OR⁴, -C(O)R⁶, -C(O)OR⁴, -O-C(O)R⁶, -NR⁴R⁵ or halogen;
R⁴ and R⁵ are each, independently of one another, hydrogen or unsubstituted C₁-C₄-alkyl;
R⁶ is unsubstituted C₁-C₁₀-alkyl.

2. The 2,6-BAMP derivative according to claim 1, wherein the 2,6-BAMP derivative is defined according to the general formula (Ia), 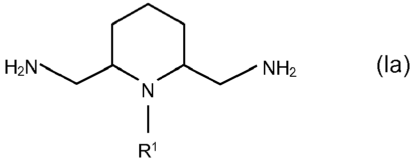 where:
R¹ is unsubstituted or at least monosubstituted C₁-C₁₀-alkyl, unsubstituted or at least monosubstituted C₂-C₁₀-alkenyl or unsubstituted or at least monosubstituted aryl,
where the substituents are selected from the group consisting of C₁-C₄-alkyl, aryl², -OR⁴, -NR⁴R⁵ or halogen,
and aryl² can in turn be at least monosubstituted by C₁-C₄-alkyl, -OR⁴, -NR⁴R⁵ or halogen;
R⁴ and R⁵ are each, independently of one another, hydrogen or unsubstituted C₁-C₄-alkyl.

3. The 2,6-BAMP derivative according to claim 1 or 2, wherein:
R¹ is unsubstituted or at least monosubstituted C₁-C₄-alkyl and the substituent is -NR⁴R⁵;
R⁴ and R⁵ are each, independently of one another, hydrogen or unsubstituted C₁-C₄-alkyl.

4. A process for preparing a 2,6-bis(aminomethyl)piperidine derivative (2,6-BAMP derivative) of the general formula (I), 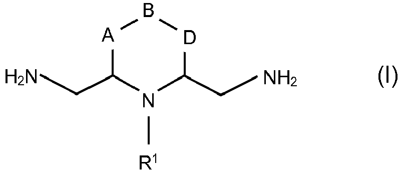 wherein the 2,6-BAMP derivative is obtained from the corresponding 2,6-dicyanopiperidine derivative (2,6-DCP derivative) of the general formula (II) or (IIa) 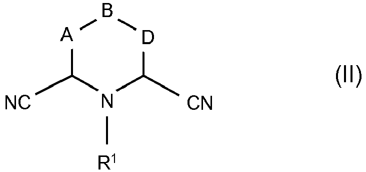 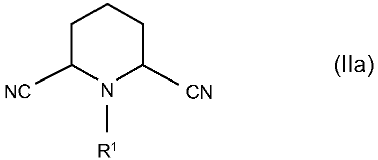 by hydrogenation in the presence of a catalyst, where:
A, B and D are each, independently of one another, CH₂, CHR² or CR²R³;
R¹ is hydrogen, unsubstituted or at least monosubstituted C₁-C₃₀-alkyl, unsubstituted or at least monosubstituted C₂-C₁₀-alkenyl or unsubstituted or at least monosubstituted aryl,
where the substituents are selected from the group consisting of C₁-C₄-alkyl, aryl², -OR⁴, -C(O)R⁶, -C(O)OR⁴, -O-C(O)R⁶, -NR⁴R⁵ or halogen,
and aryl² can in turn be at least monosubstituted by C₁-C₄-alkyl, -OR⁴, -C(O)R⁶, -C(O)OR⁴, -O-C(O)R⁶, -NR⁴R⁵ or halogen;
R² and R³ are each, independently of one another, unsubstituted or at least monosubstituted C₁-C₁₀-alkyl or unsubstituted or at least monosubstituted aryl,
where the substituents are selected from the group consisting of C₁-C₄-alkyl, -OR⁴, -C(O)R⁶, -C(O)OR⁴, -O-C(O)R⁶, -NR⁴R⁵ or halogen;
R⁴ and R⁵ are each, independently of one another, hydrogen or unsubstituted C₁-C₄-alkyl;
R⁶ is unsubstituted C₁-C₁₀-alkyl.

5. The process according to claim 4, wherein a Raney catalyst, preferably a Raney nickel catalyst or a Raney cobalt catalyst, in particular a Raney cobalt catalyst, which comprises at least one of the elements Fe, Ni or Cr as promoter is used as catalyst.

6. The process according to claim 4 or 5, wherein the hydrogenation is carried out in a solvent which is preferably an amide, an aromatic hydrocarbon, an alcohol, an amine, an ester or an ether and is in particular tetrahydrofuran.

7. The process according to any of claims 4 to 6, wherein the hydrogenation is carried out at a pressure from 80 to 200 bar and/or at a temperature from 90 to 130°C.

8. The process according to any of claims 4 to 7, wherein the 2,6-DCP derivative used for the hydrogenation is prepared by reaction of i) glutaraldehyde or a glutaraldehyde derivative with ii) hydrogen cyanide (HCN) and iii) an appropriate amine of the formula R¹NH₂.

9. The use of a 2,6-BAMP derivative according to the definition of the general formula (I) in claim 4 as hardener for epoxy resins, as intermediate in the preparation of diisocyanates, as starter in the preparation of polyetherols and/or as monomer for polyamide production.

10. A diisocyanate of the general formula (III), 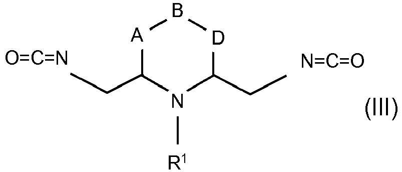 where:
A, B and D are each, independently of one another, CH₂, CHR² or CR²R³;
R¹ is hydrogen, unsubstituted or at least monosubstituted C₁-C₃₀-alkyl, unsubstituted or at least monosubstituted C₂-C₁₀-alkenyl or unsubstituted or at least monosubstituted aryl,
where the substituents are selected from the group consisting of C₁-C₄-alkyl, aryl², -OR⁴, -C(O)R⁶, -C(O)OR⁴, -O-C(O)R⁶, -NR⁴R⁵ or halogen,
and aryl² can in turn be at least monosubstituted by C₁-C₄-alkyl, -OR⁴, -C(O)R⁶, -C(O)OR⁴, -O-C(O)R⁶, -NR⁴R⁵ or halogen;
R² and R³ are each, independently of one another, unsubstituted or at least monosubstituted C₁-C₁₀-alkyl or unsubstituted or at least monosubstituted aryl,
where the substituents are selected from the group consisting of C₁-C₄-alkyl, -OR⁴, -C(O)R⁶, -C(O)OR⁴, -O-C(O)R⁶, -NR⁴R⁵ or halogen;
R⁴ and R⁵ are each, independently of one another, hydrogen or unsubstituted C₁-C₄-alkyl;
R⁶ is unsubstituted C₁-C₁₀-alkyl.

11. The diisocyanate according to claim 10, wherein the diisocyanate is defined according to the general formula (IIIa), 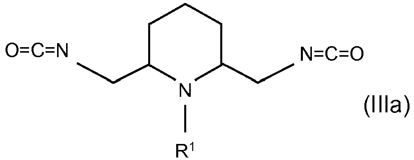 where:
R¹ is hydrogen, unsubstituted or at least monosubstituted C₁-C₁₀-alkyl or unsubstituted or at least monosubstituted aryl,
where the substituents are selected from the group consisting of C₁-C₄-alkyl, aryl², -OR⁴, -NR⁴R⁵ or halogen,
and aryl² can in turn be at least monosubstituted by C₁-C₄-alkyl, -OR⁴, -NR⁴R⁵ or halogen;
R⁴ and R⁵ are each, independently of one another, hydrogen or unsubstituted C₁-C₄-alkyl.

12. A process for preparing a diisocyanate according to claim 10 or 11, wherein a 2,6-BAMP derivative according to the definition of the general formula (II) in claim 4 is reacted with phosgene.

## Revendications

1. Dérivé de 2,6-bis-(aminométhyl)pipéridine (dérivé de 2,6-BAMP) selon la formule générale (I) 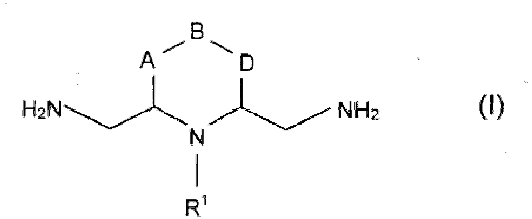 dans laquelle :
A, B et D représentent indépendamment les uns des autres CH₂, CHR² ou CR²R³ ;
R¹ représente alkyle en C₁-C₃₀ non substitué ou au moins monosubstitué, alcényle en C₂-C₁₀ non substitué ou
au moins monosubstitué, ou aryle non substitué ou au moins monosubstitué,
les substituants étant choisis dans le groupe constitué par alkyle en C₁-C₄, aryle², -OR⁴, -C(O)R⁶, - C(O)OR⁴, -O-C(O)R⁶, -NR⁴R⁵ ou halogène,
et aryle² pouvant lui-même être au moins monosubstitué avec alkyle en C₁-C₄, -OR⁴, -C(O)R⁶, - C(O)OR⁴, -O-C(O)R⁶, -NR⁴R⁵ ou halogène ;
R² et R³ représentent indépendamment l'un de l'autre alkyle en C₁-C₁₀ non substitué ou au moins monosubstitué, ou aryle non substitué ou au moins monosubstitué,
les substituants étant choisis dans le groupe constitué par alkyle en C₁-C₄, -OR⁴, -C(O)R⁶, -C(O)OR⁴, - O-C(O)R⁶, -NR⁴R⁵ ou halogène,
R⁴ et R⁵ représentent indépendamment l'un de l'autre hydrogène ou alkyle en C₁-C₄ non substitué ;
R⁶ représente alkyle en C₁-C₁₀ non substitué.

2. Dérivé de 2,6-BAMP selon la revendication 1, **caractérisé en ce que** le dérivé de 2,6-BAMP est défini selon la formule générale (Ia) 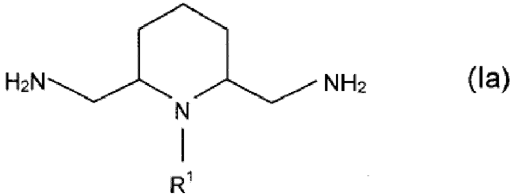 dans laquelle :
R¹ représente alkyle en C₁-C₁₀ non substitué ou au moins monosubstitué, alcényle en C₂-C₁₀ non substitué ou
au moins monosubstitué, ou aryle non substitué ou au moins monosubstitué,
les substituants étant choisis dans le groupe constitué par alkyle en C₁-C₄, aryle², -OR⁴, -NR⁴R⁵ ou halogène,
et aryle² pouvant lui-même être au moins monosubstitué avec alkyle en C₁-C₄, -OR⁴, -NR⁴R⁵ ou halogène ;
R⁴ et R⁵ représentent indépendamment l'un de l'autre hydrogène ou alkyle en C₁-C₄ non substitué.

3. Dérivé de 2,6-BAMP selon la revendication 1 ou 2, dans lequel :
R¹ représente alkyle en C₁-C₄ non substitué ou au moins monosubstitué, et le substituant est -NR⁴R⁵;
R⁴ et R⁵ représentent indépendamment l'un de l'autre hydrogène ou alkyle en C₁-C₄ non substitué.

4. Procédé de fabrication d'un dérivé de 2,6-bis-(aminométhyl)pipéridine (dérivé de 2,6-BAMP) selon la formule générale (I) 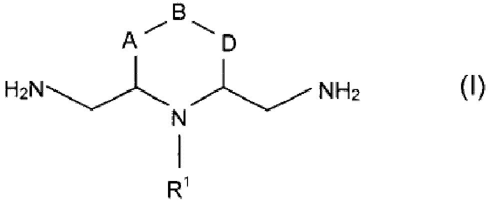 **caractérisé en ce que** le dérivé de 2,6-BAMP est obtenu à partir du dérivé de 2,6-dicyanopipéridine (dérivé de 2,6-DCP) correspondant selon la formule générale (II) ou (IIa) 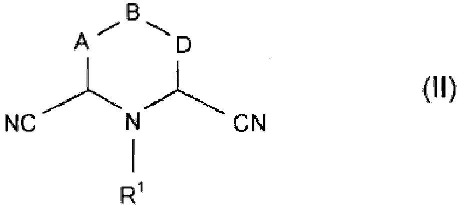 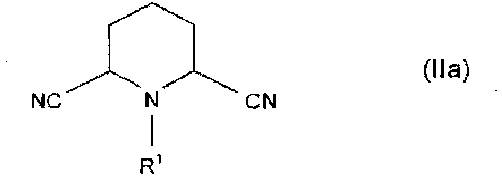 par hydrogénation en présence d'un catalyseur, dans lesquelles :
A, B et D représentent indépendamment les uns des autres CH₂, CHR² ou CR²R³ ;
R¹ représente hydrogène, alkyle en C₁-C₃₀ non substitué ou au moins monosubstitué, alcényle en C₂-C₁₀ non substitué ou au moins monosubstitué, ou aryle non substitué ou au moins monosubstitué,
les substituants étant choisis dans le groupe constitué par alkyle en C₁-C₄, aryle², -OR⁴, -C(O)R⁶, - C(O)OR⁴, -O-C(O)R⁶, -NR⁴R⁵ ou halogène,
et aryle² pouvant lui-même être au moins monosubstitué avec alkyle en C₁-C₄, -OR⁴, -C(O)R⁶, - C(O)OR⁴, -O-C(O)R⁶, -NR⁴R⁵ ou halogène ;
R² et R³ représentent indépendamment l'un de l'autre alkyle en C₁-C₁₀ non substitué ou au moins monosubstitué, ou aryle non substitué ou au moins monosubstitué,
les substituants étant choisis dans le groupe constitué par alkyle en C₁-C₄, -OR⁴, -C(O)R⁶, -C(O)OR⁴, - O-C(O)R⁶, -NR⁴R⁵ ou halogène,
R⁴ et R⁵ représentent indépendamment l'un de l'autre hydrogène ou alkyle en C₁-C₄ non substitué ;
R⁶ représente alkyle en C₁-C₁₀ non substitué.

5. Procédé selon la revendication 4, **caractérisé en ce qu'**en tant que catalyseur, un catalyseur de Raney est utilisé, de préférence un catalyseur de Raney à base de nickel ou un catalyseur de Raney à base de cobalt, notamment un catalyseur de Raney à base de cobalt, qui contient en tant que promoteur au moins un des éléments Fe, Ni ou Cr.

6. Procédé selon la revendication 4 ou 5, **caractérisé en ce que** l'hydrogénation est réalisée dans un solvant, le solvant étant de préférence un amide, un hydrocarbure aromatique, un alcool, une amine, un ester ou un éther, le solvant étant notamment le tétrahydrofurane.

7. Procédé selon l'une quelconque des revendications 4 à 6, **caractérisé en ce que** l'hydrogénation est réalisée à une pression de 80 à 200 bar et/ou à une température de 90 à 130 °C.

8. Procédé selon l'une quelconque des revendications 4 à 7, **caractérisé en ce que** le dérivé de 2,6-DCP utilisé pour l'hydrogénation est fabriqué par mise en réaction i) de glutaraldéhyde ou d'un dérivé de glutaraldéhyde avec ii) du cyanure d'hydrogène (HCN) et iii) une amine correspondante de formule R¹NH₂.

9. Utilisation d'un dérivé de 2,6-BAMP selon la définition de la formule générale (I) dans la revendication 4 en tant que durcisseur pour résines époxy, en tant que produit intermédiaire lors de la fabrication de diisocyanates, en tant que démarreur lors de la fabrication de polyétherols et/ou en tant que monomère pour la fabrication de polyamides.

10. Diisocyanate selon la formule générale (III) 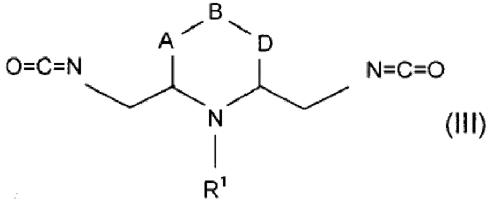 dans laquelle :
A, B et D représentent indépendamment les uns des autres CH₂, CHR² ou CR²R³ ;
R¹ représente hydrogène, alkyle en C₁-C₃₀ non substitué ou au moins monosubstitué, alcényle en C₂-C₁₀ non substitué ou au moins monosubstitué, ou aryle non substitué ou au moins monosubstitué,
les substituants étant choisis dans le groupe constitué par alkyle en C₁-C₄, aryle², -OR⁴, -C(O)R⁶, - C(O)OR⁴, -O-C(O)R⁶, -NR⁴R⁵ ou halogène,
et aryle² pouvant lui-même être au moins monosubstitué avec alkyle en C₁-C₄, -OR⁴, -C(O)R⁶, - C(O)OR⁴, -O-C(O)R⁶, -NR⁴R⁵ ou halogène ;
R² et R³ représentent indépendamment l'un de l'autre alkyle en C₁-C₁₀ non substitué ou au moins monosubstitué, ou aryle non substitué ou au moins monosubstitué,
les substituants étant choisis dans le groupe constitué par alkyle en C₁-C₄, -OR⁴, -C(O)R⁶, -C(O)OR⁴, - O-C(O)R⁶, -NR⁴R⁵ ou halogène,
R⁴ et R⁵ représentent indépendamment l'un de l'autre hydrogène ou alkyle en C₁-C₄ non substitué ;
R⁶ représente alkyle en C₁-C₁₀ non substitué.

11. Diisocyanate selon la revendication 10, **caractérisé en ce que** le diisocyanate est défini selon la formule générale (IIIa) 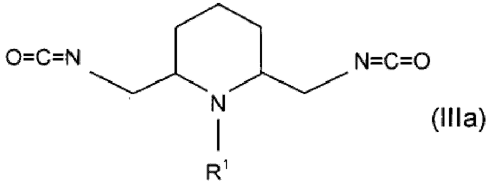 dans laquelle :
R¹ représente hydrogène, alkyle en C₁-C₁₀ non substitué ou au moins monosubstitué, ou aryle non substitué ou au moins monosubstitué,
les substituants étant choisis dans le groupe constitué par alkyle en C₁-C₄, aryle², -OR⁴, -NR⁴R⁵ ou halogène,
et aryle² pouvant lui-même être au moins monosubstitué avec alkyle en C₁-C₄, -OR⁴, -NR⁴R⁵ ou halogène ;
R⁴ et R⁵ représentent indépendamment l'un de l'autre hydrogène ou alkyle en C₁-C₄ non substitué.

12. Procédé de fabrication d'un diisocyanate selon la revendication 10 ou 11, **caractérisé en ce qu'**un dérivé de 2,6-BAMP selon la définition de la formule générale (II) dans la revendication 4 est mis en réaction avec du phosgène.
